# EUROPEAN PATENT SPECIFICATION

(11) **EP 1 567 155 B1**
(45) Date of publication and mention of the grant of the patent: **27.10.2010**
(21) Application number: 03790358.0
(22) Date of filing: 05.12.2003
(51) Int. Cl.: C12N 5/0784, A61K 31/44, C12N 5/00, C12N 5/02

(54) **ADMINISTRATION OF DENDRITIC CELLS PARTIALLY MATURED IN VITRO FOR THE TREATMENT OF TUMORS**
VERABREICHUNG TEILGEREIFTER DENDRITISCHER ZELLEN IN VITRO ZUR BEHANDLUNG VON TUMOREN
ADMINISTRATION DE CELLULES DENDRITIQUES PARTIELLEMENT MATURES IN VITRO POUR LE TRAITEMENT DE TUMEURS

(30) Priority: 06.12.2002 US 431267 P
(43) Date of publication of application: 31.08.2005
(60) Divisional application: 10176476.9
(73) Proprietor: NorthWest Biotherapeutics, Inc., Bothell WA 98011-9501 (US)
(72) Inventor: BOSCH, Marnix, L., Medina, WA 98039 (US)
(74) Representative: Gowshall, Jonathan Vallance
(86) International application number: PCT/US2003/038672
(87) International publication number: WO 2004/053072

(56) References cited:
- WO-A-00/47719
- WO-A-03/010292
- WO-A-2004/076651
- US-B1- 6 558 951
- TRIMBLE LINDA ET AL: "Maturation of dendritic cells for cancer immunotherapy, using inactivated BCG and interferon gamma: Induction of a vigorous Th1 response" PROCEEDINGS OF THE AMERICAN ASSOCIATION FOR CANCER RESEARCH ANNUAL MEETING, vol. 43, March 2002 (2002-03), page 680, XP001208099 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X
- NESTLE F.O.: 'Dendritic cell vaccination for cancer therapy' ONCOGENE vol. 19, 2000, pages 6673 - 6679, XP002986077
- TSUJI S. ET AL: 'Maturation of human dendritic cells by cell wall skeleton of Mycobacterium bovis Bacillus Calmette-Guerin: involvement of toll like receptors' INFECTION AND IMMUNITY vol. 68, 2000, pages 6883 - 6890, XP000995248
- KADOWAKI N. ET AL: 'Distinct CpG DNA and polyinosinic-polycytidilic acid double stranded RNA, resspectively stimulate CD 11c- type 2 dendritic cell precursors and CD 11+ dendritic cells to produce typeI IFN' THE JOURNAL OF IMMUNOLOGY vol. 166, 2001, pages 2291 - 2295, XP002218081
- TJOA B.A. ET AL: 'Dendritic cell-based immunotherapy for prostate cancer' CA CANCER J CLIN. vol. 49, 1999, pages 117 - 128, XP002986078
- GRANUCCI F. ET AL: EUR J IMMUNOL, vol. 31, 2001, pages 2539-2546,
- RAMONER R. ET AL: J UROLOGY, vol. 159, 1998, pages 1488-1492,
- SCHLIENGER K. ET AL: BLOOD, vol. 96, 2000, pages 3490-3498,
- LIN C.-L. ET AL: CANCER RESEARCH, vol. 62, 2002, pages 6952-6958,
- SHANKAR G. ET AL: J TRANSLATIONAL MEDICINE, vol. 1, 2003, pages 7-18,
- LUTZ M.B. ET AL: TRENDS IMMUNOL, vol. 23, 2002, pages 445-449,
- MELERO I. ET AL: GENE THERAPY, vol. 6, 1999, pages 1779-1784,
- REA D. ET AL: J VIROL, vol. 73, 1999, pages 10245-10253,
- HIRSCHOWITZ E.A. ET AL: GENE THERAPY, vol. 7, 2000, pages 1112-1120,

## Description

### BACKGROUND OF THE INVENTION

Dendritic cells (DCs) are recognized as the vehicle of choice for active immunotherapy of cancer. Animal experiments have demonstrated the potential of DC based immunotherapy in both protecting mice from tumor formation and eliminating established tumors. These successes have been at least partially duplicated in humans in small clinical trials. The transition from small safety- or proof-of-concept trials to larger trials in which activity or efficacy can be demonstrated has been hindered by the laborious and cumbersome nature of DC preparation (see below). As a consequence, few companies have been interested in developing DC-based cancer vaccines despite the large potential therapeutic of such products.

Intratumoral (IT) injection of DCs is a special form of DC-based immunotherapy. Upon injection, the DCs take up antigen from apoptotic or dying tumor cells, and present the antigen to T cells after migration to the lymph nodes. Indeed it was found that the efficacy of such treatments in animal models correlates with the degree of apoptosis in the tumor (Candido et al., Cancer Res. 61:228-236, 2001), which suggests that this approach is fully compatible with treating tumors with chemotherapeutic agents or radiation prior to the injection of DCs. In addition, several groups have demonstrated that such combination therapy is particularly effective against established tumors (Nikitina et al., Int. J. Cancer 94:825-833, 2001; Tanaka et al., Int. J. Cancer 101:265-269, 2002; Tong et al., Cancer Res. 61:7530-7535,2001).

Since the tumor cells are the source of antigen, IT injection foregoes the need for both the selection and manufacturing of tumor antigens as they are currently used in most *in vitro* DC based therapy approaches. Selection of a tumor antigen is often driven by the need for companies to have a proprietary position and the few tumor antigens identified to date have yet to be proven to provide significant clinical benefit. In addition, the use of such tumor antigens often results in a monovalent vaccine, which can lose its effectiveness if the tumor cells down regulate the expression of the antigen used in immunization. Of course, the need to manufacture the tumor antigen under conditions required under Good Manufacturing Practices (GMP) adds additional cost to classical DC-based immunization methods.

IT injection of DCs subjects the dendritic cells to an immunosuppressive tumor environment. Tumors are known to produce cytokines that inactivate the DCs or that have the ability to skew T cell response toward a less effective Th2-type response. Several groups have used genetic modification of DCs to attempt to overcome these suppressive effects, especially through the production of the cytokine Interleukin 12 (IL-12; Nishioka et al., Cancer Res. 59:4035-4041, 1999; Melero et al., Gene Therapy 6:1779-1784, 1999) or expression of CD40 ligand (Kikuchi et al., Blood 96:91-99, 2000). The encouraging results described by these groups further demonstrate the viability of IT injection of DCs as a therapeutic approach.

Triozzi et al. (Cancer 89:2647-2654, 2000) describe IT injection of DCs in patients with metastatic melanoma or breast cancer. They obtained tumor regression in 4 patients with melanoma and in two patients with breast carcinoma. Biopsies of the regressing lesions demonstrated infiltrating T cells, suggesting that the DC had indeed activated an immune response against the tumor cells. Overall these data demonstrated that IT injection of DCs was feasible in humans, and could provide significant clinical benefit. However, significant down regulation of MHC class II antigens and of the B7-2 costimulatory molecule on injected DCs has been observed. Down regulation of these critical molecules would be expected to reduce the immunostimulatory potential of the DCs, but as provided in the present invention this can be avoided through partial maturation of the DCs prior to administration.

DCs exist in peripheral tissues in an immature form, ready to take up and process antigen. It is this immature cell that is most closely mimicked by the DCs generated from monocytes in the presence of GM-CSF and IL-4. Various stimuli can initiate the maturation of DCs, during which process the cells lose their capacity to take up antigen efficiently, and gain their T cell stimulatory functions. This process is complex and at least *in vitro* can take up to 48 hours to complete. One other consequence of maturation is a change in the migratory properties of the cells. For example, maturation induces several chemokine receptors, including CCR7, which direct the cells to the T cell regions of draining lymph nodes, where the mature DCs activate T cells against the antigens that are presented on the DC surface in the context of class I and class II MHC molecules.

Romaner R. et al: J Urol, 159: 1488-1492, 1998, discloses maturation of dendritic cells with BCG in a 48 hour contact period. The dendritic cells are used for *in vitro* T-cell proliferation assays.

Lutz MB et al; Trends Immunol, 23: 445-449, 2002, discloses a semi-mature state of dendritic cells which are tolerogenic, or which require further activation, for example through a secondary LPS stimulus *in vitro* or which might respond to further stimuli after subcutaneous injection.

The induction of tolerance has been linked to the cross-presentation of (self) antigens by DCs (Kurts et al., J. Exp. Med. 186:239-245, 1997), and the current prevailing hypothesis posits that immature DC continually present antigens to the immune system to maintain tolerance (Kurts et al., J. Exp. Med. 184:923-930, 1996), suggesting that maturation of DCs *in vivo* is required for efficient immunostimulation. It has now become apparent that DC maturation can result in either immunostimulatory or immunosuppressive DCs (Chakraborty et al., Clin. Immunol. 94:88-98, 1999). The precise nature of the maturation stimuli that produce either outcome has not been elucidated, but it is generally accepted that immunostimulatory DCs produce IL-12 and express CD40 ligand (Albert et al., Nature Immunol. 2:988-989, 2001; Lanzavecchia, Haematologica 84 Suppl. EHA 4:23-25, 1999). Therefore, partially matured DC should be used for IT injection, especially if the stimuli used for maturation of immature dendritic cells are known to result in expression of CD40 ligand and in the production of IL-12.

### BRIEF SUMMARY OF THE INVENTION

The present invention provides a composition according to the claims. The disclosure provides a method for producing an anti-tumor immune response comprising the administration of a cell population comprising dendritic cells that have been partially matured *in vitro* such that the partially matured dendritic cells retain the ability to take up tumor antigen and can induce an immune response subsequent to administration to an individual. The method provides for the efficient up take and processing of tumor antigens within the tumor and tumor bed in spite of the overall immunosuppressive environment within the tumor. This immunosuppressive environment would be expected to interfere with dendritic cell function by negatively impacting on their immunostimulatory properties. Further, mature dendritic cells would not be administered to tumors because the cells do not efficiently process antigen.

Administration of the partially matured dendritic cells can be directly into an existing tumor. Further, administration can be into the tumor bed, the tissue region in and around a tumor either before or after treatment, *i*.*e*., surgical removal or resection of the tumor, prior to, simultaneous with, or subsequent to chemotherapy, radiation therapy, or combinations thereof, and the like. The partially matured dendritic cells can also be administered to a lymph node or lymphatic region that directly drains the tumor or a surrounding tumor region. In addition the partially matured dendritic cells can be administered into a circulatory vessel or into a lymphatic region that delivers blood or lymph directly into the tumor or tumor bed, or the organ afflicted by or with the tumor. Still further, the partially matured dendritic cells can be administered generally into the circulatory or lymphatic system such that the cells will be delivered into the tumor region.

Dendritic cells or precursors thereof, useful in the present invention can be obtained from, for example, skin, spleen, bone marrow, thymus, lymph nodes, umbilical cord blood, or peripheral blood, and the like. Further, the dendritic cells can be obtained from an individual to be treated or from a healthy individual HLA-matched to the individual to be treated. The dendritic cells or precursors thereof can be cryopreserved prior to being contacted with a maturation agent and formulated into a composition for administration to an individual.

The dendritic cells used in the methods of the disclosure are partially matured *in vitro.* Suitable agents for the induction of dendritic cell maturation include, for example, but are not limited to, Bacillus Calmette-Guerin (BCG), interferon γ (IFNγ), lipopolysaccharide (LPS), tumor necrosis factor α (TNFα), an imidazoquinoline compound, *e*.*g*., a imidazoquinoline-4-amine compound, such as 4-amino-2-ethoxymethyl-α,α-dimethyl-1H-imidazol[4,5-c]quinolin-1-ethanol (designated R848) or 1-(2-methylpropyl)-1H-imidazo[4,5-c]quinolin-4-amine, and their derivatives (WO 00/47719), a synthetic double stranded polyribonucleotide, *e*.*g*., poly[I]:poly[C(12)U], and the like, agonists of a Toll-like receptor (TLR), such as TLR-3, TLR-4, TLR-7 and/or TLR-9, a sequence of nucleic acids containing unmethylated CpG motifs known to induce the maturation of DC, and the like, or a combination thereof.

BCG as used in the present invention can comprise whole BCG, cell wall constituents of BCG, BCG-derived lipoarabidomannans, or any other BCG components. In certain embodiments, the BCG is heat-inactivated BCG or formalin-treated BCG. combinations of BCG and IFNγ are used for the partial maturation of the dendritic cells, although as an alternative disclosure BCG can be used alone. An effective amount of BCG is typically about 10⁵ to 10⁷ cfu per milliliter of tissue culture media and an effective amount of IFNγ is typically about 100 to about 1000 Units per milliliter of tissue culture media. In another embodiment the dendritic cell activating agent is the imidazoquinoline R848. Typically, an effective amount of R848, a TLR-4 agonist, that is effective is about 1 to about 50 µg/ml, more typically 5 to 10 µg/ml of tissue culture media is used. The imidazoquinoline can be used alone or it can be combined with, for example, an effective amount of BCG and/or IFNγ.

The present disclosure also comprises compositions, the composition comprise dendritic cells partially matured *in vitro* in the presence of a dendritic cell maturation agent combined with a physiologically acceptable carrier, buffer and/or excipient for administration to an individual with a tumor. Partially mature dendritic cells used in the compositions of the disclosure typically have up-regulated expression of co-stimulatory molecules such as, but not limited to CD80, CD86 or CD54. The cells may or may not express the cell surface marker CD83, but still can uptake and process antigen. The composition typically will comprise about 10² to about 10¹⁰, but can comprise more, partially matured dendritic cells. Subsequent to partial maturation the dendritic cells can be cryopreserved and stored for a period of time prior to administration to an individual. The cells used to produce the partially matured dendritic cells that comprise the compositions of the present invention can be isolated from the patient having the tumor or, the partially matured dendritic cells can be produce from cells that have been isolated from an individual that has been HLA matched to the patient.

### DETAILED DESCRIPTION OF THE INVENTION

Dendritic cells are a diverse population of antigen presenting cells found in a variety of lymphoid and non-lymphoid tissues. (*See* Liu, Cell 106:259-62 (2001); Steinman, Ann. Rev Immunol. 9:271-96 (1991)). Dendritic cells include lymphoid dendritic cells of the spleen, Langerhans cells of the epidermis, and veiled cells in the blood circulation. Collectively, dendritic cells are classified as a group based on their morphology, high levels of surface MHC-class II expression, and absence of certain other surface markers expressed on T cells, B cells, monocytes, and natural killer cells. In particular, monocyte-derived dendritic cells (also referred to as monocytic dendritic cells) usually express CD11c, CD80, CD86, and are HLA-DR⁺, but are CD14⁻.

In contrast, monocytic dendritic cell precursors (typically monocytes) are usually CD14⁺. Monocytic dendritic cell precursors can be obtained from any tissue where they reside, particularly lymphoid tissues such as the spleen, bone marrow, lymph nodes and thymus. Monocytic dendritic cell precursors also can be isolated from the circulatory system. Peripheral blood is a readily accessible source of monocytic dendritic cell precursors. Umbilical cord blood is another source of monocytic dendritic cell precursors. Monocytic dendritic cell precursors can be isolated from a variety of organisms in which an immune response can be elicited. Such organisms include animals, for example, including humans, and non-human animals, such as, primates, mammals (including dogs, cats, mice, and rats), birds (including chickens), as well as transgenic species thereof.

In certain embodiments, the monocytic dendritic cell precursors and/or immature dendritic cells can be isolated from a healthy subject or from a subject in need of immunostimulation, such as, for example, a cancer patient or other subject for whom cellular immunostimulation can be beneficial or desired (*i.e*., a subject having a bacterial or viral infection, and the like). Dendritic cell precursors and/or immature dendritic cells also can be obtained from an HLA-matched healthy individual for partial activation and administration to an HLA-matched subject in need of immunostimulation.

### Dendritic Cell Precursors and Immature Dendritic Cells

Methods for isolating cell populations enriched for dendritic cell precursors and immature dendritic cells from various sources, including blood and bone marrow, are known in the art. For example, dendritic cell precursors and immature dendritic cells can be isolated by collecting heparinized blood, by apheresis or leukapheresis, by preparation of buffy coats, rosetting, centrifugation, density gradient centrifugation (*e*.*g*., using Ficoll^{®} (such as FICOLL-PAQUE^{①}), PERCOLL^{®} (colloidal silica particles (15-30 nm diameter) coated with non-dialyzable polyvinylpyrrolidone (PVP)), sucrose, and the like), differential lysis of cells, filtration, and the like. In certain embodiments, a leukocyte population can be prepared, such as, for example, by collecting blood from a subject, defribrinating to remove the platelets and lysing the red blood cells. Dendritic cell precursors and immature dendritic cells can optionally be enriched for monocytic dendritic cell precursors by, for example, centrifugation through a PERCOLL^{®} gradient, antibody panning, and the like.

Dendritic cell precursors and immature dendritic cells optionally can be prepared in a closed, aseptic system. As used herein, the terms "closed, aseptic system" or "closed system" refer to a system in which exposure to non-sterile, ambient, or circulating air or other non-sterile conditions is minimized or eliminated. Closed systems for isolating dendritic cell precursors and immature dendritic cells generally exclude density gradient centrifugation in open top tubes, open air transfer of cells, culture of cells in tissue culture plates or unsealed flasks, and the like. In a typical embodiment, the closed system allows aseptic transfer of the dendritic cell precursors and immature dendritic cells from an initial collection vessel to a sealable tissue culture vessel without exposure to non-sterile air.

Another reported method for isolating dendritic cell precursors is to use a commercially treated plastic substrate (*e*.*g*., beads or magnetic beads) to selectively remove adherent monocytes and other "non-dendritic cell precursors." (*See, e*.*g*., U.S. Patent Nos. 5,994,126 and 5,851,756.) The adherent monocytes and non-dendritic cell precursors are discarded while the non-adherent cells are retained for *ex vivo* culture and maturation. In another method, apheresis cells were cultured in plastic culture bags to which plastic, *i*.*e*., polystyrene or styrene, microcarrier beads were added to increase the surface area of the bag. Cells were cultured for a sufficient period of time for certain cells to adhere to the beads and the non-adherent cells were washed from the bag. (Maffei, et al., Transfusion 40:1419-1420 (2000); WO 02/44338).

In certain other embodiments, monocytic dendritic cell precursors are isolated by adherence to a monocyte-binding substrate, as disclosed in WO 03/010292. For example, a population of leukocytes (*e*.*g*., isolated by leukapheresis) can be contacted with a monocytic dendritic cell precursor adhering substrate. When the population of leukocytes is contacted with the substrate, the monocytic dendritic cell precursors in the leukocyte population preferentially adhere to the substrate. Other leukocytes (including other potential dendritic cell precursors) exhibit reduced binding affinity to the substrate, thereby allowing the monocytic dendritic cell precursors to be preferentially enriched on the surface of the substrate.

Suitable substrates include, for example, those having a large surface area to volume ratio. The substrate can be, for example, a particulate or fibrous substrate. Suitable particulate substrates include, for example, glass particles, plastic particles, glass-coated plastic particles, glass-coated polystyrene particles, and other beads suitable for protein absorption. Fibrous substrates suitable for use in the present invention include microcapillary tubes and microvillous membranes, and the like. The particulate or fibrous substrate usually allows the adhered monocytic dendritic cell precursors to be eluted without substantially reducing the viability of the adhered cells. A particulate or fibrous substrate can be substantially non-porous to facilitate elution of monocytic dendritic cell precursors or dendritic cells from the substrate. A "substantially non-porous" substrate is a substrate in which at least a majority of pores present in the substrate are smaller than the cells to minimize entrapping cells in the substrate.

Adherence of the monocytic dendritic cell precursors to the substrate can optionally be enhanced by the addition of a binding media. Suitable binding media include monocytic dendritic cell precursor culture media (*e*.*g*., AIM-V^{®}, RPMI 1640, DMEM, X-VIVO 15^{®}, and the like) supplemented, individually or in any combination, with for example, cytokines (*e*.*g*., Granulocyte/Macrophage Colony Stimulating Factor (GM-CSF), Interleukin 4 (IL-4),or Interleukin 13 (IL-13)), blood plasma, serum (*e*.*g*., human serum, such as autologous or allogenic sera), purified proteins, such as serum albumin, divalent cations (*e*.*g*., calcium and/or magnesium ions) and other molecules that aid in the specific adherence of monocytic dendritic cell precursors to the substrate, or that prevent adherence of non-monocytic dendritic cell precursors to the substrate. In certain embodiments, the blood plasma or serum can be heated-inactivated. The heat-inactivated plasma can be autologous or heterologous to the leukocytes.

Following adherence of monocytic dendritic cell precursors to the substrate, the non-adhering leukocytes are separated from the monocytic dendritic cell precursor/substrate complexes. Any suitable means can be used to separate the non-adhering cells from the complexes. For example, the mixture of the non-adhering leukocytes and the complexes can be allowed to settle, and the non-adhering leukocytes and media decanted or drained. Alternatively, the mixture can be centrifuged, and the supernatant containing the non-adhering leukocytes decanted or drained from the pelleted complexes.

In another method, monocytic dendritic cell precursors can be isolated from a cell population enriched in leukocytes prepared by the use of a tangential flow filtration device as described in International Patent Application No. PCT/US03/19428, filed June 19, 2003 and having publication no. WO 2004/000444. A tangential flow filtration device useful for the isolation of a cell population enriched in monocytic dendritic cell precursors can comprise a remover unit having a cross-flow chamber, a filtrate chamber and a filter disposed therebetween. The filter is in fluid communication on one side, the retentate surface, with the cross-flow chamber, and on the other side, the filtrate surface, with the filtrate chamber. The cross-flow chamber has an inlet adapted to introduce a sample of blood constituents comprising leukocytes into the cross-flow chamber and parallel to the retentate surface of the filter. An outlet is also provided in the cross-flow chamber centrally disposed in a portion of the chamber opposite the retentate surface of the filter. The filter suitable for use in the tangential flow filtration device typically has an average pore size ranging from about 1 to about 10 microns. The filter can have an average pore size of about 3 to about 7 microns. A means for providing a predetermined input rate of the sample into the inlet of the cross-flow chamber and a means for controlling a filtration rate of filtrate through the filter and into the filtrate chamber can also be included. The filtration rate controlling means limits the rate of filtration to less than the unopposed filtration rate for the filter. The sample comprising blood constituents can be provided by a source device such as a leukapheresis device or a container comprising a sample collected from a leukapheresis device.

Monocytic dendritic cell precursors and cell populations enriched for the precursors can be cultured *ex vivo* for differentiation, and partial maturation and/or expansion. As used herein, isolated immature dendritic cells, dendritic cell precursors, and other cells, refers to cells that, by human hand, exist apart from their native environment, and are therefore not a product of nature. Isolated cells can exist in purified form, in semipurified form, or in a non-native environment. Briefly, *ex vivo* differentiation typically involves culturing monocytic dendritic cell precursors, or populations of cells having dendritic cell precursors, in the presence of one or more differentiation agents. Suitable differentiating agents can include, for example, cellular growth factors (*e*.*g*., cytokines such as (GM-CSF), Interleukin 4 (IL-4), Interleukin 7 (IL-7), Interleukin 13 (IL-13), and/or combinations thereof). In certain embodiments, the monocytic dendritic cells precursors are differentiated to form monocyte-derived immature dendritic cells.

The dendritic cell precursors can be cultured and differentiated in suitable culture conditions. Suitable tissue culture media include, but are not limited to, AIM-V^{®}, RPMI 1640, DMEM, X-VIVO 15^{®}, and the like. The tissue culture media can be supplemented with serum, amino acids, vitamins, cytokines, such as GM-CSF and/or IL-4, IL-7, IL-13, divalent cations, and the like, to promote differentiation of the cells. In certain embodiments, the dendritic cell precursors can be cultured in serum-free media. The culture conditions can optionally exclude any animal-derived products. A typical cytokine combination used with dendritic cell culture medium comprises about 500 units/ml each of GM-CSF and IL-4, IL-7, or IL-13. Dendritic cell precursors, when differentiated to form immature dendritic cells, are phenotypically similar to skin Langerhans cells. Immature dendritic cells typically are CD14⁻ and CD11c⁺, express low levels of CD86 and CD83, and are able to capture soluble antigens via specialized endocytosis.

Dendritic cell maturation agents of the disclosure can include, for example, but are not limited to, BCG, IFNγ, LPS, TNFα, an imidazoquinoline compound, *e*.*g*., a imidazoquinoline-4-amine compound, such as 4-amino-2-ethoxymethyl-α,α-dimethyl-1H-imidazol[4,5-c]quinolin-1-ethanol (designated R848) or 1-(2-methylpropyl)-1H-imidazo[4,5-c]quinolin-4-amine, and their derivatives (WO 00/47719), a synthetic double stranded polyribonucleotide, *e*.*g*., poly[I]:poly[C(12)U], and the like, agonists of a Toll-like receptor (TLR), such as TLR-3, TLR-4, TLR-7 and/or TLR-9, a sequence of nucleic acids containing unmethylated CpG motifs known to induce the maturation of DC, and the like, or any combination thereof. Effective amounts of BCG typically range from about 10⁵ to 10⁷ cfu per milliliter of tissue culture media. Effective amounts of IFNγ typically range from about 100-1000 U per milliliter of tissue culture media. Bacillus Calmette-Guerin (BCG) is an avirulent strain of *M. bovis.* As used herein, BCG refers to whole BCG as well as cell wall constituents, BCG-derived lipoarabidomannans, and other BCG components. BCG is optionally inactivated, such as heat-inactivated BCG, formalin-treated BCG, and the like. An effective amount of an imidazoquinoline compound, *e*.*g*., a imidazoquinoline-4-amine compound, such as 4-amino-2-ethoxymethyl-α,α-dimethyl-1H-imidazol[4,5-c]quinolin-1-ethanol (designated R848) can be about 1 to about 50 µg/ml of culture medium, more typically 5 to about 10 µg/ml of culture media is used. The imidazoquinoline compound can be used alone or can be combined with, for example BCG and/or IFNγ, or an additional TLR agonist.

The immature DCs are typically contacted with effective amounts of the dendritic cell maturation agent, such as BCG and IFNγ, for about one hour to about 10 hours. Typically at least a 24 hour incubation period is required for complete maturation. More typically 48 to about 72 hours incubation. The immature dendritic cells can be cultured and partially matured in suitable maturation culture conditions. Suitable tissue culture media include AIM-V^{®}, RPMI 1640, DMEM, X-VIVO 15^{®}, and the like. The tissue culture media can be supplemented with amino acids, vitamins, cytokines, such as GM-CSF and/or IL-15, divalent cations, and the like, to promote maturation of the cells. A typical cytokine combination is about 500 units/ml of GM-CSF and 100 ng/ml of IL-15.

Partial maturation of immature dendritic cells can be monitored by methods known in the art for dendritic cells. Cell surface markers can be detected in assays familiar to the art, such as flow cytometry, immunohistochemistry, and the like. The cells can also be monitored for cytokine production (*e*.*g*., by ELISA, another immune assay, or by use of an oligonucleotide array). In DCs cultured and partially matured according to the present invention in the presence of a dendritic cell maturation agent, such as GM-CSF and IL-4, the levels of phosphorylated JAK2 (janus activated kinase 2) can be measured to indicate the initiation of maturation by methods well known in the art. The induction of the expression of cell surface markers and cytokines, as well as the phosphorylation of signaling molecules, *e*.*g*., jak2, is also known as an indicator that dendritic cells are conditioned for induction of an immune response once the cells have been administered to an individual.

The immature dendritic cells are subject to maturation only for a time period to partially mature the dendritic cells. Fully mature DCs lose the ability to take up antigen and display up-regulated expression of costimulatory cell surface molecules and various cytokines. Specifically, mature DCs express higher levels of MHC class I and II antigens than immature dendritic cells, and mature dendritic cells are generally identified as being CD80⁺, CD83⁺, CD86⁺, and CD14⁻. Greater MHC expression leads to an increase in antigen density on the DC surface, while up regulation of costimulatory molecules CD80 and CD86 strengthens the T cell activation signal through the counterparts of the costimulatory molecules, such as CD28 on the T cells. Partially mature dendritic cells as used in the present invention typically comprise those dendritic cells that once exposed to a dendritic cell maturation agent demonstrate an up-regulation of expression of a co-stimulating molecule including, but not limited to, CD80, CD86 and/or CD54. The cell may or may not express CD83, but maintains the ability to uptake and process antigen. Further, the partially mature dendritic cells may produce TNF-α, IL-6, IL-10 and or IL-12 which are not typically produced by immature dendritic cells.

Fully mature dendritic cells are not preferred for the present invention because they do not efficiently process antigen. Further, immature dendritic cells as used in prior methods are not desired because the immunosuppressive environment typically found within a tumor, including substantial concentrations of cytokines known to prevent the processing of antigen by immature dendritic cells. In the present invention, partial maturation of the immature dendritic cells down regulates cytokine receptors on the surface of the cell rendering them less sensitive or responsive to any immunosuppressive effects of cytokines present in the intratumoral space and provides for cells that can efficiently process tumor antigens present within the intratumoral space. Once the partially mature dendritic cells have matured within the tumor as measured by the production of, for example, TNF-α and IL-12, and the expression of the chemokine receptor CCR7, the cells can migrate to the lymph nodes where the cells will contact T cells to up regulate the immune response to the tumor antigens.

According to yet another aspect of the invention, DCs can be preserved, *e*.*g*., by cryopreservation either before maturation or following partial maturation prior to administration to a patient. Cryopreservation agents which can be used include but are not limited to dimethyl sulfoxide (DMSO), glycerol, polyvinylpyrrolidone, polyethylene glycol, albumin, dextran, sucrose, ethylene glycol, i-erythritol, D-ribitol, D-mannitol, D-sorbitol, i-inositol, D-lactose, choline chloride, amino acids, methanol, acetamide, glycerol monoacetate, and inorganic salts. A controlled slow cooling rate can be critical. Different cryoprotective agents and different cell types typically have different optimal cooling rates. The heat of fusion phase where water turns to ice typically should be minimal. The cooling procedure can be carried out by use of, *e*.*g*., a programmable freezing device or a methanol bath procedure. Programmable freezing apparatuses allow determination of optimal cooling rates and facilitate standard reproducible cooling. Programmable controlled-rate freezers such as Cryomed or Planar permit tuning of the freezing regimen to the desired cooling rate curve.

After thorough freezing, monocytic precursor cells, immature DCs or partially mature DCs can be rapidly transferred to a long-term cryogenic storage vessel. In a typical embodiment, samples can be cryogenically stored in liquid nitrogen (-196° C) or its vapor (-165° C). Considerations and procedures for the manipulation, cryopreservation, and long term storage of hematopoietic stem cells, particularly from bone marrow or peripheral blood, is largely applicable to the cells of the invention. Such a discussion can be found, for example, in the following references: Taylor et al., Cryobiology 27:269-78 (1990); Gorin, Clinics in Haematology 15:19-48 (1986); Bone-Marrow Conservation, Culture and Transplantation, Proceedings of a Panel, Moscow, Jul. 22-26, 1968, International Atomic Energy Agency, Vienna, pp. 107-186.

Frozen cells are preferably thawed quickly (*e*.*g*., in a water bath maintained at 37°-41° C) and chilled immediately upon thawing. It may be desirable to treat the cells in order to prevent cellular clumping upon thawing. To prevent clumping, various procedures can be used, including but not limited to the addition before and/or after freezing of DNase (Spitzer et al., Cancer 45: 3075-85 (1980)), low molecular weight dextran and citrate, hydroxyethyl starch (Stiff et al., Cryobiology 20: 17-24 (1983)), and the like. The cryoprotective agent, if toxic in humans, should be removed prior to therapeutic use of the thawed partially matured DCs. One way in which to remove the cryoprotective agent is by dilution to an insignificant concentration. Once frozen partially matured DCs have been thawed and recovered, they can be administered as described herein with respect to non-frozen partially matured DCs.

### In vivo Administration of Partially Matured Dendritic Cells

Methods are provided for administration of partially mature dendritic cells, or a cell population enriched and containing such cells, to a subject having a tumor. In certain embodiments, such methods are performed by obtaining dendritic cell precursors or immature dendritic cells, differentiating and partially maturing those cells in the presence of a dendritic cell maturation agent, such as BCG and IFNγ, or any other maturation agent such as those listed above. The partially matured dendritic cells can be formulated with physiologically acceptable carriers, excipients, buffers and/or diluents using methods and compositions well known to the skilled artisan. The partially mature dendritic cells can be administered directly to a subject in need of immunostimulation. Typically, about 10² to about 10¹⁰ cells are suspended in a pharmaceutically acceptable carrier. The cells are injected either into the tumor directly or into a region near to, adjacent to, or in circulatory or lymphatic contact with the tumor or tumor bed to ensure that the cells have access to the tumor antigens.

For example, but not by limitation, the cells can be administered directly into a tumor, into the tumor bed subsequent to surgical removal or resection of the tumor, peri-tumorily, into a draining lymph node in direct contact with the tumor, into a blood vessel or lymph duct leading into, or feeding a tumor or organ afflicted by the tumor, *e*.*g*., the portal vein or a pulmonary vein or artery, and the like. The administration of the partially mature dendritic cells of the disclosure can be either simultaneous with or subsequent to other treatments for the tumor, such as chemotherapy or radiation therapy. Further, the partially mature dendritic cells of the invention can be co-administered with another agent, which agent acts as an adjuvant to the maturation of the dendritic cell and/or the processing of antigen within the tumor or region near or adjacent to the tumor. In addition, the dendritic cells can also be formulated or compounded into a slow release matrix for implantation into a region in or around the tumor or tumor bed such that cells are slowly released into the tumor, or tumor bed, for contact with the tumor antigens.

Partially mature dendritic cells of the present invention can be administered by any means appropriate for the formulation and mode of administration. For example, the cells can be combined with a pharmaceutically acceptable carrier and administered with a syringe, a catheter, a cannula, and the like. As above, the cells can be formulated in a slow release matrix. When administered in this fashion, the formulation can be administered by a means appropriate for the matrix used. Other methods and modes of administration applicable to the present disclosure are well known to the skilled artisan.

Compositions of the present invention can be used by themselves in the treatment of an individual. In addition, the compositions can be used in combination with any other method to treat a tumor. For example, the methods of the present invention can be used in combination with surgical resection of a tumor, chemotherapy (cytotoxic drugs, apoptotic agents, antibodies, and the like), radiation therapy, cryotherapy, brachytherapy, immune therapy (administration of antigen specific mature activated dendritic cells, NK cells, antibodies specific for tumor antigens, etc.), and the like. Any and all of these methods can also be used in any combination. Combination treatments can be concurrent or sequential and can be administered in any order as determined by the treating physician.

In another embodiment, the dendritic cells and the recipient subject have the same MHC (HLA) haplotype. Methods of determining the HLA haplotype of a subject are known in the art. In a related embodiment, the partially mature dendritic cells are allogenic to the recipient subject. The allogenic cells are typically matched for at least one MHC allele (*e*.*g*., sharing at least one but not all MHC alleles). In a less typical embodiment, the dendritic cells and the recipient subject are all allogeneic with respect to each other, but all have at least one MHC allele in common.

### Examples

The following examples are provided merely as illustrative of various aspects of the present invention and should not be construed to limit the invention in any way. Any examples that do not fall wherein the scope of the claims are provided for comparison purposes only.

### Example 1

In the present example, monocytic dendritic cell precursors, isolated by tangential flow filtration, were cultured at a concentration of 10⁶ monocytes/ml in X-VIVO 15^{®} supplemented with 2% human serum albumin (HSA) and 500 U/ml of GM-CSF in either flasks or cell bags. After 5 days, the DCs from the flasks and the bags were either left untreated (iDCs) or were partially matured for 4 hours in the presence of BCG (1:400 dilution) and interferon γ (IFN-γ, 500 U/ml)(pmDCs). The cells were collected, washed and mixed with an equal number of fluorescently labeled irradiated PKH67-A549 tumor cells for 48 hours. Following the incubation, the cells were washed, stained with a phycoerythrin (PE) labeled monoclonal antibody specific for CD11c, and analyzed by flow cytometry. The values provided in Table 1 represent the percentage of CD11c⁺ cells within the DC gate that were also positive for labeled PKH67 antigen (MFI = mean fluorescent intensity). Controls included DCs mixed with PKH67-A549 cells just prior to analysis.

**Table 1.**

| Treatment | % CD11c⁺PKH67⁺ Cells | MFI |
|---|---|---|
| iDCs-flask | 87.6 | 235.2 |
| pmDCs-flask | 90.7 | 610.2 |
| iDCs-bag | 72.8 | 403.5 |
| pmDCs-bag | 91.3 | 557.5 |

These data demonstrate that the partially matured DCs were better at antigen uptake in this assay than immature dendritic cells, as measured by either the percentage of cells that take up antigen or by the amount of material picked up by the tumor cells.

In another example, a leukapheresis was carried out on a patient subsequent to surgery for removal of a tumor growth. Monocytes were purified from the leukapheresis product by tangential flow filtration. The purified monocytes were differentiated as described above and partially matured by exposure to heat killed and formalin fixed BCG (5 x 10⁶ cfu/ml) IFNγ (500 U/ml). The partially matured dendritic cells were formulated for administration and injected into the tumor bed of the individual, under ultrasound guidance. Dendritic cells isolated from the individual were found to be capable of inducing a tumor specific cytotoxic T cell response when measured *in vitro.* Further, it was found that the time to tumor recurrence was prevented or substantially delayed.

### Example 2

In this example human colon cancer cells were grafted into mice to form tumor xenografts by well known methods. After the tumors were established, animals were divided into groups and the animals of each group were administered either placebo, immature dendritic cells or partially matured dendritic cells into the tumor. The size of the tumor was subsequently measured and compared for each group.

Briefly, human CT26 colon carcinoma tumors were established in female Balb/c mice by injecting 100,000 tumor cells in the right flank under the skin. Treatment was initiated on day 15, at which time all mice had palpable tumors with tumor sizes ranging between 10 mm² and 45 mm².

Dendritic cells were prepared from the femoral bone marrow of female Balb/c mice following standard protocols. Briefly, bone marrow cells following red cell lysis were incubated with murine GM-CSF (200 U/ml) and IL-4 (200 U/ml) in RPMI 1640 supplemented with 10% fetal bovine serum for 13 - 14 days, with addition of fresh media with cytokines after 3 and 7 days. To obtain partially matured dendritic cells, the cells were exposed to a 400-fold dilution of inactivated Bacillus Calmette-Guerin (BCG) that had activity of 0.5 - 1 x 10⁶ colony forming units per milliliter prior to inactivation, and to 500 units of murine interferon gamma (IFNγ) per milliliter. The cells were incubated fro 8 hours and then layered over 14.5% metrizamide and centrifuged for 15 minutes at 4 °C and 1750 rpm. The interface was collected and the cells were washed and viably frozen in 10% dimethyl sulfoxide (DMSO) in liquid nitrogen storage. Following thawing at 37 °C, the cells were washed twice and resuspended at a concentration of 1 million cells per 50 microliters.

The tumor bearing mice were treated with cyclophosphamide (50 mg/kg) intraperitoneally on day 15 and 17. Dendritic cell injection in the tumor (either mock injection of 50 µl of phosphate buffered saline, immature DC or DC matured for 8 hours) was performed on days 16, 17, 18 and 21). Tumor growth was measured every other day for 60 days.

In the group treated intratumorally with phosphate buffered saline, all 4 mice had tumors greater than 100 mm² on or before day 40. In contrast, the mice treated intratumorally with immature DC had reduced tumor growth and 2 of 4 mice had tumors smaller than 100 mm² on day 40. Three of the 4 mice treated with partially matured DC had tumors smaller than 100 mm² on day 40, and two of those animals had no palpable tumors on day 50.

### Example 3

In this example human colon cancer cells were injected into mice to from tumor xenografts by well known methods. Subsequent to the first injection of tumor cells the animals were administered a second dose of tumor cells. After 15 days, animals were divided into groups and the animals of each group were administered either placebo, immature dendritic cells or partially matured dendritic cells into the tumor. The size of the tumor was subsequently measured and compared for each group.

Briefly, human CT26 colon carcinoma tumors were established in female Balb/c mice by injecting 100,000 tumor cells in the right flank under the skin, followed by injection of 100,000 tumor cells in the left flank on day 3. Treatment was initiated on day 15, at which time all mice had palpable right-flank tumors with tumor sizes ranging between 10 mm² and 45 mm².

Dendritic cells were prepared from the femoral bone marrow of female Balb/c mice following standard protocols. Briefly, bone marrow cells following red cell lysis were incubated with murine GM-CSF (200 U/ml) and IL-4 (200 U/ml) in RPMI 1640 supplemented with 10% fetal bovine serum for 13 - 14 days, with addition of fresh media with cytokines after 3 and 7 days. To obtain partially matured dendritic cells, the cells were exposed to a 400-fold dilution of inactivated Bacillus Calmette-Guerin (BCG) that had activity of 0.5 - 1 x 10⁶ colony forming units per milliliter prior to inactivation, and to 500 units of murine interferon gamma per milliliter. The cells were then layered over 14.5% metrizamide and centrifuged for 15 minutes at 4 °C and 1750 rpm. The interface was collected and the cells were washed and viably frozen in 10% dimethyl sulfoxide (DMSO) in liquid nitrogen storage. Following thawing at 37 °C, the cells were washed twice and resuspended at a concentration of 1 million cells per 50 microliters.

The tumor bearing mice were treated with cyclophosphamide (50 mg/kg) intraperitoneally on day 15 and 17. Dendritic cell injection in the right flank tumor (either mock injection of 50 µl of phosphate buffered saline, immature DC, DC matured for 8 hours or DC matured for 24 hours was performed on days 16, 17, 18 and 21). Tumor growth was measured every other day for 60 days.

In the group treated intratumorally with phosphate buffered saline, all 5 mice had right-flank tumors greater than 100 mm² before day 35, and 3/5 animals had left-flank tumors > 100 mm² on or before day 40. In contrast, the mice treated intratumorally (right side only) with immature DC had reduced tumor growth: one mouse had no palpable tumor on either side at day 60, and 4 of 5 mice had tumors < 100 mm² on both sides at day 40. Three of the 5 mice treated with 8 hour partially matured DC had completely resolved both tumors by day 50. In the group treated with DC that had been partially matured for 24 hours, tumor growth was reduced even further. Three of 5 mice completely resolved the tumors on both sides by day 50, one animal completely resolved the left-flank tumor, and partially controlled the right-flank tumor (< 100 mm² on day 40) and one animal partially controlled both tumors (< 50 mm² on both sides at day 40).

### Example 4

In this example, human tumor cells were used to form xenograph tumors in mice as described above. The differentiated immature dendritic cells were partially matured by exposing the cells to the dendritic cell maturation agent imidazoquinoline R848 or R848 combined with BCG and IFNγ. The partially matured dendritic cells, immature dendritic cells and a placebo were administered into the tumor and the size of the tumors in each group were compared.

Briefly, tumors were established in mice as in example 2. Dendritic cells were prepared as in examples 2 and 3, but partial maturation of the cells was achieved by exposing the cells to 5 µg/ml of the immune response modifier (dendritic cell maturation agent) R848, or 5 µg/ml of the immune response modifier R848 together with BCG and interferon gamma at the concentrations as set forth in examples 2 and 3, for 8 hours.

The treatment schedule was identical to example 3. None of the mice injected intratumorally with phosphate buffered saline controlled tumor growth. One of five mice treated with DC partially matured with R848 alone fully resolved tumor growth, as did 3 of 5 mice treated intratumorally with DC partially matured with R848 with BCG and IFNγ. Nine mice from examples 3 and 4 that had completely resolved their tumors were challenged approximately 30 days after tumor resolution in the right flank with 1 million tumor cells. None of the animals showed detectable tumor growth demonstrating immunological protection against the CT26 tumor cells.

### Example 5

In this example the dendritic cells prepared in the examples above were characterized for the expression of surface molecules. Briefly, murine dendritic cells, prepared as in experiments 2 - 4, were characterized for expression of cell surface molecules after staining with fluorescent antibodies and flow cytometric analysis. The results are presented as the percentage of cells positive for the phenotypic surface markers and the mean fluorescence intensity for certain surface markers in Tables 2 and 3.

**Table 2.**

| | Percent positive cells | | | | | |
|---|---|---|---|---|---|---|
| treatment | CD11c | MHC-II | CD80 | CD86 | CD54 | CD40 |
| Immature | 66.9 | 68.1 | 62.2 | 28.1 | 62.6 | 1.4 |
| R848 (8 hrs) | 60.7 | 76.4 | 73.4 | 31.3 | 96.6 | 1.5 |
| BCG/IFN (8 hrs) | 59.5 | 85.5 | 84.9 | 66.0 | 96.0 | 2.0 |
| BCG/IFN/R848 (8 hrs) | 58.4 | 88.8 | 82.4 | 61.8 | 96.6 | 2.6 |
| BCG/IFN (24 hrs) | 66.3 | 91.5 | 90.0 | 84.4 | 95.3 | 3.3 |

**Table 3.**

| | Mean fluorescence intensity | | | | |
|---|---|---|---|---|---|
| treatment | MHC-II | CD80 | CD86 | CD54 | CD40 |
| Immature | 395.7 | 50.0 | 30.7 | 53.8 | 3.0 |
| R848 (8 hrs) | 515.5 | 54.7 | 30.0 | 90.0 | 8.0 |
| BCG/IFN (8 hrs) | 751.1 | 79.1 | 74.6 | 120.3 | 7.2 |
| BCG/IFN/R848 (8 hrs) | 757.8 | 79.3 | 66.4 | 132.9 | 13.9 |
| BCG/IFN (24 hrs) | 876.8 | 97.9 | 99.0 | 126.9 | 11.4 |

### Example 7

A patient diagnosed with a solid tumor is treated with chemotherapy, radiation therapy, cryotherapy, or brachytherapy and subsequently with partially matured dendritic cells administered intratumorally. Following treatment, the tumor is resolved and the patient is protected from tumor recurrence.

The previous examples are provided to illustrate, but not to limit, the scope of the claimed inventions. Other variants of the inventions will be readily apparent to those of ordinary skill in the art and encompassed by the appended claims.

## Claims

1. A composition comprising dendritic cells partially matured *in vitro* in the presence of a dendritic cell maturation agent combined with a pharmaceutically acceptable carrier for *in vivo* administration, wherein the partially matured dendritic cells demonstrate up regulation of expression of co-stimulating molecule CD80, CD86 and/or CD54, and phosphorylation of JAK2, and maintain the ability to uptake and process antigen, for use in the treatment of tumors **characterized in that** the composition is for administration directly into the tumor or into a region near to, adjacent to, or in circulatory or lymphatic contact with the tumor, or tumor bed; and wherein the dendritic cell maturation agent is Bacillus Calmette-Guerin (BCG) and interferon γ (IFNγ); BCG, INFγ and imidazoquinoline R848; or R848 alone.

2. The composition according to claim 1, wherein the dendritic cells are obtained from skin, spleen , bone marrow, thymus, lymph nodes, umbilical cord blood, or peripheral blood.

3. The composition according to claim 2, wherein the dendritic cells are obtained from the individual to be treated.

4. The composition according to claim 2, wherein the dendritic cells are obtained from a healthy individual HLA-matched to the individual to be treated.

5. The composition according to claim 1, wherein the BCG comprises whole BCG, cell wall constituents of BCG, BCG-derived lipoarabidomannans, BCG components, or the BCG is heat-inactivated BCG or formalin-treated BCG.

6. The composition according to claim 1, wherein the effective amount of BCG is about 10⁵ to 10⁷ cfu per milliliter of tissue culture media, the effective amount of IFNγ is about 100 to about 1000 Units per milliliter of tissue culture media.

7. The composition according to any of claims 1 through 6, wherein the partially matured dendritic cells are administered as an adjuvant to radiation therapy, chemotherapy, or combinations thereof.

8. The composition according to any of claims 1 through 6, wherein the composition comprises about 10² to about 10¹⁰ partially matured dendritic cells.

9. The composition according to any of claims 1 through 6, wherein the partially matured dendritic cells have been cryopreserved subsequent to partial maturation.

## Patentansprüche

1. Zusammensetzung, die dendritische Retikulumzellen umfasst, die *in vitro* in Gegenwart eines Mittels zur Reifung dendritischer Retikulumzellen partiell gereift wurden, in Kombination mit einem pharmazeutisch unbedenklichen Trägerstoff zur *In*-*vivo-*Verabreichung, wobei die partiell gereiften dendritischen Retikulumzellen eine Hochregulation der Expression des kostimulierenden Moleküls CD80, CD86 und/oder CD54 und eine Phosphorylierung von JAK2 zeigen und die Fähigkeit bewahren, Antigen aufzunehmen und zu verarbeiten, zur Verwendung bei der Behandlung von Tumoren, **dadurch gekennzeichnet, dass** die Zusammensetzung zur Verabreichung direkt in den Tumor oder einen Bereich in der Nähe des Tumors, benachbart zum Tumor oder in zirkulatorischem oder lymphatischem Kontakt mit dem Tumor oder das Tumorbett ist; und wobei es sich bei dem Mittel zur Reifung dendritischer Retikulumzellen um Bacillus Calmette-Guérin (BCG) und Interferon-γ (IFN-γ); BCG, INF-γ und Imidazochinolin R848; oder R848 für sich handelt.

2. Zusammensetzung nach Anspruch 1, wobei die dendritischen Retikulumzellen aus Haut, Milz, Knochenmark, Thymus, Lymphknoten, Nabelschnurblut oder peripherem Blut bezogen werden.

3. Zusammensetzung nach Anspruch 2, wobei die dendritischen Retikulumzellen von der zu behandelnden Person bezogen werden.

4. Zusammensetzung nach Anspruch 2, wobei die dendritischen Retikulumzellen von einer gesunden Person bezogen werden, deren HLA mit denen der zu behandelnden Person abgeglichen wurden.

5. Zusammensetzung nach Anspruch 1, wobei das BCG kompletten BCG, Zellwandbestandteile von BCG, von BCG abgeleitete Lipoarabinomannane, BCG-Bestandteile umfasst oder es sich bei dem BCG um hitzeinaktivierten BCG oder mit Formalin behandelten BCG handelt.

6. Zusammensetzung nach Anspruch 1, wobei die wirksame Menge von BCG etwa 10⁵ bis 10⁷ KBE pro Milliliter Gewebekulturmedium beträgt, wobei die wirksame Menge von IFN-γ etwa 100 bis etwa 1000 Einheiten pro Milliliter Gewebekulturmedium beträgt.

7. Zusammensetzung nach einem der Ansprüche 1 bis 6, wobei die partiell gereiften dendritischen Retikulumzellen als ein Adjuvans zu Strahlentherapie, Chemotherapie oder Kombinationen davon verabreicht werden.

8. Zusammensetzung nach einem der Ansprüche 1 bis 6, wobei die Zusaimnensetzung etwa 10² bis etwa 10¹⁰ partiell gereifte dendritische Retikulumzellen umfasst.

9. Zusammensetzung nach einem der Ansprüche 1 bis 6, wobei die partiell gereiften dendritischen Retikulumzellen nach der partiellen Reifung kryokonserviert wurden.

## Revendications

1. Une composition comprenant des cellules dendritiques partiellement matures ***in vitro*** en présence d'un agent de maturation des cellules dendritiques combiné à un transport pharmaceutiquement acceptable pour l'administration ***in vivo,*** dans laquelle les cellules dendritiques partiellement matures présentent une régulation positive de l'expression des molécules co-stimulantes CD80, CD86 et/ou CD54, et la phosphorylation de la JAK2, et conservent la capacité de capturer et traiter les antigènes, pour l'utilisation dans le traitement de tumeurs **caractérisées** dans lesquels la composition est destinée à être administrée directement dans la tumeur ou dans une région voisine, adjacente, ou au contact circulatoire ou lymphatique de la tumeur, ou dans le lit de la tumeur ; et dans laquelle l'agent de maturation des cellules dendritiques est le Bacille Calmette-Guerin (BCG) et l'interféron γ (IFN γ) ; le BCG, l'INFγ et l'imidazoquinoline R848 ; ou le R848 seul.

2. La composition conforme à la revendication 1, dans laquelle les cellules dendritiques sont obtenues à partir de peau, rate , moelle osseuse, thymus, ganglions lymphatiques, sang du cordon ombilical, ou sang périphérique.

3. La composition conforme à la revendication 2, dans laquelle les cellules dendritiques sont obtenues à partir de l'individu à traiter.

4. La composition conforme à la revendication 2, dans laquelle les cellules dendritiques sont obtenues à partir de d'un individu sain HLA-compatible avec l'individu à traiter.

5. La composition conforme à la revendication 1, dans laquelle le BCG comprend le BCG entier, les constituants de la membrane cellulaire du BCG, les lipoarabidomannans dérivés du BCG, les composants du BCG, ou le BCG est un BCG désactivé par la chaleur ou un BCG traité par formaline.

6. La composition conforme à la revendication 1, dans laquelle la quantité effective de BCG est d'environ 10⁵ à environ 10⁷ cfu par millilitre de milieu de culture tissulaire, a quantité effective d'IFNγ est d'environ 100 à environ 1000 unités par millilitre de milieu de culture tissulaire.

7. La composition conforme à l'une quelconque des revendications 1 à 6, dans laquelle les cellules dendritiques partiellement matures sont administrées comme adjuvant à une radiothérapie, une chimiothérapie ou des combinaisons de celles-ci.

8. La composition conforme à l'une quelconque des revendications 1 à 6, dans laquelle la composition comprend environ 10² à 10¹⁰ cellules dendritiques partiellement matures.

9. La composition conforme à l'une quelconque des revendications 1 à 6, dans laquelle les cellules dendritiques partiellement matures ont été cryoconservées après maturation partielle.
